(19) Europäisches Patentamt
European Patent Office
Office européen des brevets

(11) Veröffentlichungsnummer: **0 020 970**
**A1**

(12) **EUROPÄISCHE PATENTANMELDUNG**

(21) Anmeldenummer: **80102556.0**

(22) Anmeldetag: **09.05.80**

(51) Int. Cl.³: **C 07 C 131/00**
// C07D233/61, C07D249/08, C07D413/12

(30) Priorität: **16.06.79 DE 2924401**

(43) Veröffentlichungstag der Anmeldung: **07.01.81**
**Patentblatt 81/1**

(84) Benannte Vertragsstaaten: **AT BE CH DE FR GB IT LI LU NL SE**

(71) Anmelder: **BASF Aktiengesellschaft,**
**Carl-Bosch-Strasse 38, D-6700 Ludwigshafen (DE)**

(72) Erfinder: **Buschmann, Ernst, Dr., An der Froschlache 7, D-6700 Ludwigshafen (DE)**
Erfinder: **Zeeh, Bernd, Dr., Thorwaldsenstrasse 5, D-6700 Ludwigshafen (DE)**
Erfinder: **Becker, Rainer, Dr., Sonnenwendstrasse 83A, D-6702 Bad Duerkheim 1 (DE)**
Erfinder: **Schaefer, Peter, Dr., Budapester Strasse 51, D-6700 Ludwigshafen (DE)**

(54) **Alpha-Chloracetophenonoximether und Verfahren zu ihrer Herstellung.**

(57) Die vorliegende Erfindung betrifft α-Chloracetophenonoximether der Formel I

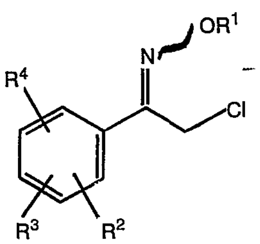

(I)

in der

$R^1$ Alkyl mit bis zu 4 Kohlenstoffatomen, Alkenyl mit bis zu 5 Kohlenstoffatomen, Alkinyl mit bis zu 4 Kohlenstoffatomen, Cycloalkyl mit bis zu 6 Kohlenstoffatomen, wobei der Alkyl-, Alkenyl-, Alkinyl- und der Cycloalkylrest jeweils durch 1 bis 3 Halogenatome substituiert sein können, gegebenenfalls durch Halogen, Alkyl oder Trifluormethyl einfach oder mehrfach im Arylrest substituiertes Aralkyl, gegebenenfalls durch Alkyl oder Halogen substituiertes Heteroarylalkyl sowie Cyanmethyl oder Alkoxycarbonylalkyl und

$R^2$, $R^3$, $R^4$ unabhängig voneinander Wasserstoff, Halogen, Alkyl oder Alkoxy mit jeweils bis zu 4 Kohlenstoffatomen, Alkylthio mit bis zu 6 Kohlenstoffatomen, gegebenenfalls durch Halogen substituiertes Aryl oder Aralkyl, Aryloxy oder Arylthio bedeuten und zwei der Reste $R^2$, $R^3$, $R^4$ zusammen einen alicyclischen oder heterocyclischen Ring bilden können, an den ein weiterer aromatischer Ring ankondensiert sein kann, sowie ein Verfahren zu ihrer Herstellung durch Umsetzung von Phenacylchloriden mit Hydroxylaminen. Die α-Chloracetophenonoximether sind wertvolle Zwischenprodukte für die Synthese von fungiziden α-Azolylacetophenonoximethern.

EP 0 020 970 A1

# α-Chloracetophenonoximether und Verfahren zu ihrer Herstellung

Die vorliegende Erfindung betrifft α-Chloracetophenonoximether und ein Verfahren zu ihrer Herstellung.

α-Bromacetophenonoximether sind bekannt; sie können durch photochemische Bromierung der entsprechenden Acetophenonoximether mit N-Bromsuccinimid erhalten werden (J. Org. Chem. 36, 3467-3469 (1971)).

Die α-Chloracetophenonoximether haben die Formel I

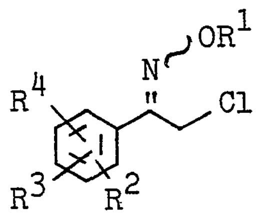

(I),

in der

$R^1$ Alkyl mit bis zu 4 Kohlenstoffatomen, Alkenyl mit bis zu 5 Kohlenstoffatomen, Alkinyl mit bis zu 4 Kohlenstoffatomen, Cycloalkyl mit bis zu 6 Kohlenstoffatomen, wobei der Alkyl-, Alkenyl-, Alkinyl- und der Cycloalkylrest jeweils durch 1 bis 3 Halogenatome substituiert sein können, gegebenenfalls durch Halogen, Alkyl oder Trifluormethyl einfach oder mehrfach im Arylrest substituiertes Aralkyl, gegebenenfalls durch Alkyl oder Halogen substituiertes Heteroarylalkyl sowie Cyanmethyl oder Alkoxycarbonylalkyl und

$R^2$, $R^3$, $R^4$ unabhängig voneinander Wasserstoff, Halogen, Alkyl oder Alkoxy mit jeweils bis zu 4 Kohlenstoffatomen, Alkylthio mit bis zu 6 Kohlenstoffatomen, gegebenenfalls durch Halogen substituiertes Aryl oder Aralkyl, Aryloxy oder Arylthio bedeuten und zwei der Reste $R^2$, $R^3$, $R^4$ zusammen einen alicyclischen oder heterocyclischen Ring

bilden können, an den ein weiterer aromatischer Ring ankondensiert sein kann.

In der Formel I steht $R^1$ für Alkyl mit bis zu 4 Kohlenstoffatomen, wie Methyl, Ethyl, n-Propyl, i-Propyl oder einen Butylrest, für Alkenyl mit bis zu 5 Kohlenstoffatomen, wie Allyl, Methallyl, Crotyl, 3-Methyl-buten-2-yl, für Alkinyl mit bis zu 4 Kohlenstoffatomen, wie Propargyl, Butin-2-yl, für Cycloalkyl mit bis zu 6 Kohlenstoffatomen, wie Cyclohexyl, Cyclopentyl. Diese Alkyl-, Alkenyl-, Alkinyl- und Cycloalkylreste können jeweils durch 1 bis 3 Halogenatome substituiert sein. $R^1$ in Formel I steht außerdem für Aralkyl, das gegebenenfalls im Arylrest einfach oder mehrfach durch Halogen, Alkyl mit 1 bis 4 Kohlenstoffatomen, vorzugsweise Methyl, oder Trifluormethyl substituiert ist, wie Benzyl, Phenethyl, 4-Chlorbenzyl, 4-Brombenzyl, 4-Fluorbenzyl, 4-Methylbenzyl, 4-Trifluormethylbenzyl, 3-Chlorbenzyl, 2,4-Dichlorbenzyl, 3,4-Dichlorbenzyl, 2,6-Dichlorbenzyl, für gegebenenfalls durch Alkyl mit 1 bis 4 Kohlenstoffatomen, insbesondere Methyl oder Ethyl, oder durch Halogen, insbesondere Chlor, substituiertes Heteroarylalkyl, wie gegebenenfalls substituierte 1,2-Oxazolyl-5-methylreste, beispielsweise 3-Ethyl-1,2-oxazolyl-5-methyl, 3-Methyl-1,2-oxazolyl-5-methyl, 2-Thiophenmethyl, 3-Thiophenmethyl, für Cyanmethyl oder für Alkoxycarbonylalkyl, wie Methoxycarbonylmethyl, Ethoxycarbonylmethyl.

Als Substituenten $R^2$, $R^3$ und $R^4$ in Formel I kommen Wasserstoff, Halogen, wie Fluor, Chlor oder Brom, Alkyl mit 1 bis 4 Kohlenstoffatomen, wie Methyl, Ethyl, n-Propyl, i-Propyl, tert.-Butyl, Alkoxy mit 1 bis 4 Kohlenstoffatomen, wie Methoxy, Ethoxy, Propoxy oder Butoxy, Alkylthio mit 1 bis 6 Kohlenstoffatomen, wie Methylthio, Ethylthio, n-Butylthio, n-Propylthio, Isobutylthio, n-Hexyl-

thio, gegebenenfalls durch Halogen substituierten Aryl oder Aralkyl, wie Phenyl, Benzyl, 4-Chlorphenyl, 4-Chlorbenzyl, 2,4-Dichlorbenzyl, Aryloxy oder Arylthio, wie Phenoxy, Phenylthio, in Betracht.

Zwei der Substituenten $R^2$, $R^3$ und $R^4$ können außerdem einen alicyclischen oder heterocyclischen Ring mit 5 oder 6 Ringgliedern bilden, an den ein weiterer aromatischer Ring ankondensiert sein kann, so daß beispielsweise gemeinsam mit dem Phenylring des Acetophenons folgende Reste gebildet werden können: Dibenzofurylreste, Dibenzothienyl.

Weiterhin wurde gefunden, daß man die neuen α-Chloracetophenonoximether der Formel I durch Umsetzung eines Phenacylchlorids der Formel II

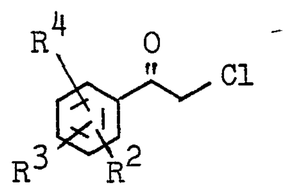 (II),

in der
$R^2$, $R^3$, $R^4$ die obengenannten Bedeutungen haben, mit einem Hydroxylamin der Formel III

$$H_2NOR^1 \qquad \text{(III)},$$

in der
$R^1$ die obengenannten Bedeutungen hat, oder einem Säureadditionssalz eines Hydroxylamins der Formel III erhält.

Überraschenderweise reagieren die Hydroxylamine der Formel III mit α-Chloracetophenon nicht wie Amine unter Substitution des Chlors, sondern mit der Carbonylgruppe unter Bildung einer Schiff'schen Base.

Die Umsetzung wird in einem unter den Reaktionsbedingungen inerten organischen Lösungsmittel durchgeführt. Geeignet sind chlorierte aliphatische oder aromatische Kohlenwasserstoffe, wie Chloroform, Methylenchlorid, 1,1-Dichlorethan, 1,2-Dichlorethan, Chlorbenzol, Alkohole, wie Methanol, Ethanol, Isopropanol, Nitrile, wie Acetonitril. Auch Gemische dieser Lösungsmittel können verwendet werden.

Die Umsetzung kann drucklos oder unter Druck, diskontinuierlich oder kontinuierlich, bei einer Temperatur im Bereich von 20 bis 120°C, vorzugsweise 60 bis 100°C, durchgeführt werden. Die Reaktionspartner werden dabei in stöchiometrischen Mengen oder in einem 10 bis 30 %igen molaren Überschuß an Hydroxylamin der Formel III bzw. eines entsprechenden Säureadditionssalzes eingesetzt. Die Umsetzung wird, wenn Säureadditionssalze von Hydroxylaminen eingesetzt werden, vorzugsweise in Gegenwart eines Puffers, beispielsweise Natriumacetat, Kaliumacetat, Natriumdihydrogenphosphat, durchgeführt.

Bei der Umsetzung können sowohl syn- als auch anti-Isomere gebildet werden.

Als Ausgangsverbindungen für die Herstellung der erfindungsgemäßen α-Chloracetonphenonoximether kommen beispielsweise folgende α-Chloracetophenone in Betracht: α-Chloracetophenon, 4-α-Dichloracetophenon, 2,4-α-Trichloracetophenon, α-Chlor-4-phenoxyacetophenon, 4-Brom-α-Chloracetophenon, α-Chlor-4-phenylthio-acetophenon, α-Chlor-4-isobutylthio-acetophenon, α-Chlor-4-methoxyacetophenon, α-Chlor-4-methyl-acetophenon, α-Chlor-4-ethyl-acetophenon, 3,4,α-Trichloracetophenon, 2-Chloracetyl-dibenzofuran.

Die Hydroxylamine der Formel III sind nach bekannten Methoden herstellbar (J. Keck in Methodicum Chimicum, Georg Thieme Verlag, Stuttgart 1974, Bd. 6, S. 69-72). Beispiele für Hydroxylamine der Formel III sind O-Methylhydroxylamin, O-Ethylhydroxylamin, O-Propylhydroxylamin, O-Isopropylhydroxylamin, O-Allyl-hydroxylamin, O-Crotylhydroxylamin, O-(3-Methyl-butin-2-yl)-hydroxylamin, O-Propargylhydroxylamin, O-Cyclohexylhydroxylamin, O-Benzylhydroxylamin, O-(4-Chlorbenzyl)-hydroxylamin, O-(2,4-Dichlorbenzyl)-hydroxylamin, O-(4-Brombenzyl)--hydroxylamin, O-(4-Trifluormethylbenzyl-hydroxylamin, O-(4-Methyl-benzyl)-hydroxylamin, O-(4-Fluorbenzyl)--hydroxylamin, O-(3-Chlorbenzyl)--hydroxylamin, O-(1,2--Oxazolyl-5-methyl)-hydroxylamin.

Die Hydroxylamine können in Form ihrer Säureadditionssalze eingesetzt werden, beispielsweise als Hydrobromide, Hydrochloride, O-substituierte Hydroxylammoniumnitrate, -sulfate, -phosphate, -acetate, -oxalate und -formiate.

Die erfindungsgemäßen α-Chloracetophenonoximether können entsprechend folgendem Beispiel hergestellt werden:

Beispiel 1

0,11 Mol α-Chloracetophenon, 0,11 Mol Natriumacetat und 0,12 Mol O-(4-Chlorbenzyl)-hydroxylammoniumhydrochlorid werden 200 ml Ethanol gelöst. Man kocht 6 Stunden unter Rückfluß, engt ein, nimmt mit Dichlormethan/Wasser auf, wäscht die organische Phase mehrfach mit Wasser, trocknet über Natriumsulfat und engt ein. Zurück bleibt der α-Chloracetophenonoxim-(4-chlorbenzyl)-ether (Nr. 27, Tabelle 1). Die Umsetzung verläuft fast quantitativ.

Beispiel 2

20 g 2,4,α-Trichloracetophenon und 11 g O-Benzylhydroxylamin werden in 200 ml Ethanol gelöst. Nach 24 Stunden Rühren bei Raumtemperatur zeigt das IR-Spektrum keine C=O-Bande mehr. Das Reaktionsgemisch wird eingeengt, mit Dichlormethan aufgenommen, mit Wasser gewaschen, über Natriumsulfat getrocknet und erneut eingeengt. Man erhält 2,4,α-Trichloracetophenonoxim-benzylether (Nr. 33, Tabelle 1). Die Umsetzung verläuft fast quantitativ.

In entsprechender Weise erhält man die in Tabelle 1 aufgeführten α-Chloracetophenonoximether der Formel I, zumeist als Gemische ihrer Syn- und Anti-Isomeren.

Tabelle 1

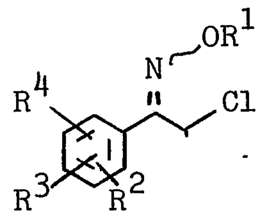

I

| Nr. | $R^1$ | $R^2$ | $R^3$ | $R^4$ | NMR ($CDCl_3$, δ-Werte) |
|---|---|---|---|---|---|
| 1 | $CH_3$ | 2-Cl | 4-Cl | H | Isomer 1: 3,85 (S, 3H), 4,37 (S, 2H) 7,18-7,42 (m, 3H)<br>Isomer 2: 4,00 (S, 3H), 4,51 (S, 2H) 7,18-7,42 (m, 3H)<br>Verhältnis: Isomer 1:Isomer 2 = 1:5 |
| 2 | n-$C_3H_7$ | 2-Cl | 4-Cl | H | Isomer 1: 0,96 (t, J=8Hz, 3H), 1,62 (quintet, J=8Hz, 2H), 3,91 (t, J=8Hz, 2H), 4,27 (S, 2H), 7,02-7,26 (m, 3H),<br>Isomer 2: 0,96 (t, J=8HZ, 3H), 1,62 (quintet, J=8Hz, 2H), 4,10 (, J=8Hz, 2H), 4,48 (S, 2H), 7,02-7,26 (m, 3H)<br>Verhältnis: Isomer 1:Isomer 2 = 1:4 |
| 3 | $C_2H_5$ | 2-Cl | 4-Cl | H | Isomer 1: 1,32 (t, J=8Hz, 3H), 4,02 (g, J=8Hz, 2H), 4,28 (S, 2H), 7,08-7,30 (m, 3H)<br>Isomer 2: 1,32 (t, J=8Hz, 3H), 4,20 (g, J=8Hz, 2H), 4,52 (S, 2H), 7,08-7,30 (m, 3H) |

Tabelle 1 (Fortsetzung)

| Nr. | $R^1$ | $R^2$ | $R^3$ | $R^4$ | NMR ($CDCl_3$, δ-Werte) |
|---|---|---|---|---|---|
| 4 | $C_2H_5$ | 4-Cl | H | H | 135 (t, J=8Hz, 3H), 4,32 (q, I=8Hz, 2H) 4,52 (s, 2H), 7,35 (d, J=7Hz, 2H), 7,67 (d, J=7Hz, 2H) |
| 5 | n-$C_3H_7$ | 4-Cl | H | H | 1,00 (t, J=8Hz, 3H) 1,75 (quintet, J=8Hz, 2H), 4,33 (t, J=8Hz, 2H), 4,62 (S, 2H), 7,55 (d, J=10 Hz, 2H), 7,84 (d', J=10Hz, 2H) |
| 6 | $CH_3$ | 4-Cl | H | H | Isomer 1: 4,02 (S, 3H), 4,52 (S, 2H) 7,58 (d, J=10Hz, 2H), 7,77 (d, J=10Hz, 2H), Isomer 2: 4,18 (S, 3H), 4,62 (S, 2H) 7,58 (d, J=10Hz, 2H), 7,77 (d, J=10Hz, 2H), Verhältnis Isomer 1:Isomer 2=1:6 |
| 7 | $CH_3$ | H | H | H | Isomer 1: 3,98 (S, 3H), 4,50 (S, 2H) 7,50-7,70 (M, 3H), 7,79-8,03 (M, 2H) Isomer 2: 4,11 (S, 3H), 4,60 (S, 2H) 7,50-7,70 (M, 3H), 7,79-8,05 (M, 2H) Verhältnis Isomer 1:Isomer 2=1:6 |
| 8 | $CH_3$ | 4-$CH_3$ | H | H | 2,20 (S, 3H), 3,86 (S, 3H), 4,32 (S, 2H), 7,03 (d, J = 8 Hz, 2H), 7,41 (d,J = 8 Hz, 2H) |
| 9 | $CH_3$ | 4-Phenyl | H | H | 4,20 (S, 3H), 4,70 (S, 2H), 7,50-8,1 (M, 9H) |

Tabelle 1 (Fortsetzung)

| Nr. | $R^1$ | $R^2$ | $R^3$ | $R^4$ | NMR ($CDCl_3$, δ-Werte) |
|---|---|---|---|---|---|
| 10 | Allyl | 2-Cl | 4-Cl | H | |
| 11 | 2,4-Dichlorbenzyl | 2-Cl | 4-Cl | H | |
| 12 | Propargyl | 2-Cl | 4-Cl | H | |
| 13 | $CH_3$ | 4-Br | H | H | |
| 14 | 4-Chlorbenzyl | 4-Br | H | H | |
| 15 | 2,4-Dichlorbenzyl | 4-Br | H | H | |
| 16 | 2,4-Dichlorbenzyl | 4-Cl | H | H | |

Tabelle 1 (Fortsetzung)

| Nr. | $R^1$ | $R^2$ | $R^3$ | $R^4$ | NMR ($CDCl_3$, $\delta$-Werte) |
|---|---|---|---|---|---|
| 17 | 4-Chlorbenzyl | 2-Cl | 4-Cl | H | |
| 18 | 2-Chlorbenzyl | 2-Cl | 4-Cl | H | |
| 19 | 2,6-Dichlorbenzyl | 2-Cl | 4-Cl | H | |
| 20 | 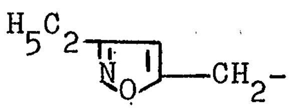 | 2-Cl | 4-Cl | H | |
| 21 | 4-Fluorbenzyl | 2-Cl | 4-Cl | H | |
| 22 | 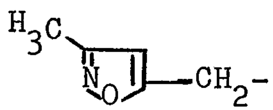 | 2-Cl | 4-Cl | H | |
| 23 | $CH_3$ | 4-Phenoxy | H | H | |

Tabelle 1 (Fortsetzung)

| Nr. | $R^1$ | $R^2$ | $R^3$ | $R^4$ | NMR ($CDCl_3$, δ-Werte) |
|---|---|---|---|---|---|
| 24 | $CH_3$ | 4-$SC_6H_5$ | H | H | |
| 25 | $CH_3$ | 4-Benzyl | H | H | |
| 26 | 4-Chlorbenzyl | 4-$CH_3$ | H | H | |
| 27 | 4-Chlorbenzyl | H | H | H | 4,62 (S, 2H), 5,53 (S, 2H), 7,37-7,86 (m, 9H) |
| 28 | 2,4-Dichlorbenzyl | 4-tert.-$C_4H_9$ | H | H | |
| 29 | $CH_2CN$ | 4-Cl | H | H | |
| 30 | $CH_2CN$ | 2-Cl | 4-Cl | H | |

Tabelle 1 (Fortsetzung)

| Nr. | $R^1$ | $R^2$ | $R^3$ | $R^4$ | NMR (CDCl$_3$, δ-Werte) |
|---|---|---|---|---|---|
| 31 | $CH_2CO_2C_2H_5$ | 2-Cl | 4-Cl | H | |
| 32 | $CH_2CO_2C_2H_5$ | 2-Cl | 4-Cl | H | |
| 33 | Benzyl | 2-Cl | 4-Cl | H | Isomer 1: 4,5 = (S, 2H), 5,24 (S, 2H), 7,12-7,47 (m, 8H),<br>Isomer 2: 4,36 (S, 2H), 5,08 S, 2H), 7,12-747 (m, 8H)<br>Verhältnis Isomer 1 : Isomer 2 = 10:1 |
| 34 | 2-Chlorbenzyl | 2-Cl | 4-Cl | H | |
| 35 | 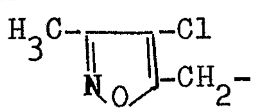 | 2-Cl | 4-Cl | H | |

Tabelle 1 (Fortsetzung)

| Nr. | $R^1$ | $R^2$ | $R^3$ | $R^4$ | NMR ($CDCl_3$, δ-Werte) |
|---|---|---|---|---|---|
| 36 | 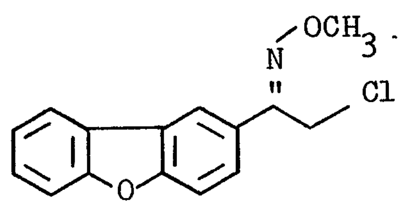 | | | | |
| 37 | $CH_3$ | 2-Cl | 4-Cl | 6-Cl | |
| 38 | Benzyl | 3-Cl | 4-Cl | H | Isomer 1: 4,50 (9, 2H), 5,80 (S, 2H), 7,52-7,24 (m, 8H); Isomer 2: 4,37 (S, 2H), 5,10 (S, 2H), 7,52-7,24 (m, 8H) Verhältnis Isomer 1 : Isomer 2 6:1 |
| 39 | 4-Chlorbenzyl | 4-Cl | H | H | 4,45 (S, 2H), 5,17 (5, 2H), 7,09 (d, J = 8 Hz, 2H) |

Tabelle 1 (Fortsetzung)

| Nr. | $R^1$ | $R^2$ | $R^3$ | $R^4$ | NMR ($CDCl_3$, -Werte) |
|---|---|---|---|---|---|
| 40 | $CH_3$ | 3-Cl | 4-Cl | H | Isomer 1: 4,04 (S, 3H), 4,42 (S, 2H), 7,36 (d, J = 8 Hz, 1H), 7,48 (dd, J = 8 + 2 Hz, 1H), 7,75 (d, J = 2 Hz, 1H); Isomer 2: 3,89 (S, 3H), 4,35 (S, 2H), 7,36 (d, 8-8 Hz, 1H), 7,48 (dd, J = 8 + 2 Hz, 1H), 7,69 (d, J = 1 Hz, 1H) |
| 41 | $CH_2CCl=CHCl$ | 2-Cl | 4-Cl | H | |
| 42 | $CH_2CCl=CHCl$ | 4-Cl | H | H | |

Die erfindungsgemäßen α-Chloracetophenonoximether der Formel I sind wertvolle Zwischenprodukte für die Synthese von fungizid wirksamen α-Azolylacetophenonoximethern der Formel IV

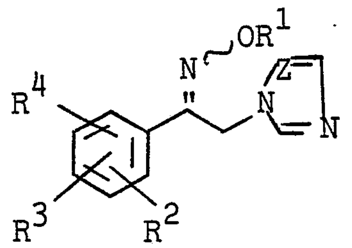

(IV),

in der
Z für CH oder N steht und $R^1$, $R^2$, $R^3$ und $R^4$ die in Formel I angegebenen Bedeutungen haben. Die fungiziden Wirkstoffe der Formel IV sind Gegenstand der DE-OS 27 23 942 und der DE-OS 26 57 578. Für ihre Herstellung wird die Umsetzung der entsprechenden Phenacylchloride oder -bromide (Halogenketone) mit 1,2,4-Triazol und Imidazol gemäß DE-OS 24 31 407 und die anschließende Oximierung und Veretherung empfohlen. Diese Synthese liefert die gewünschten Wirkstoffe nicht in der gewünschten Ausbeute und Reinheit. Demgegenüber führt die Umsetzung der aus den gut zugänglichen Phenacylchloriden erhältlichen α-Chloracetophenonoximether der Formel I mit 1,2,4-Triazol und Imidazol zu befriedigenderen Ausbeuten, abgesehen davon, daß die α-Chloracetophenonoximether für diesen Zweck nicht weiter gereinigt zu werden brauchen, das heißt, sie können ohne weitere Reinigung mit 1,2,4--Triazol oder Imidazol zu den fungiziden Wirkstoffen der Formel IV umgesetzt werden.

Die Umsetzung wird bei einer Temperatur im Bereich zwischen 20 und 120°C in einem inerten organischen Lösungsmittel durchgeführt. Geeignet sind Alkohole, wie Methanol oder Ethanol, Nitrile, wie Acetonitril, Amide, wie Dimethylformamid, Dimethylacetamid, N-Methylpyrrolidon. Auch Gemische dieser Lösungsmittel können verwendet werden.

Zur Bindung des bei der Umsetzung entstehenden Chlorwasserstoffs wird dem Reaktionsgemisch eine basische Substanz, beispielsweise ein Alkalimetallcarbonat, wie Natriumcarbonat oder Kaliumcarbonat, ein Alkoholat, wie Natriummethylat, Natriumethylat, Natriumhydrid, Butyllithium, ein Amin, wie Diisopropylethylamin, ein Lithiumamid, wie Lithiumdiisopropylamid, zugegeben. Der Chlorwasserstoff kann auch durch einen Überschuß an Imidazol oder 1,2,4-Triazol gebunden werden.

Die Reaktionspartner werden in stöchiometrischen Mengen eingesetzt. Vorzugsweise arbeitet man mit einem Überschuß an 1,2,4-Triazol bzw. Imidazol von 20 bis 200 Mol%.

Herstellung von α-(1,2,4-Triazolyl)-acetophenonoxim-O--(4-chlorbenzyl)-ethernitrat.

Ein Gemisch von 0,2 Mol 1,2,4-Triazol und 0,2 Mol Natriumcarbonat in 200 ml Ethanol wird zum Rückfluß erhitzt. Dann tropft man eine Lösung von 0,1 Mol α-Chloracetophenonoxim-O-(4-chlorbenzyl)-ether in 50 ml Ethanol zu. Nach drei Stunden Kochen unter Rückfluß wird eingeengt und mit Dichlormethan/Wasser aufgenommen. Die organische Phase wird mit Wasser gelöst. Unter Kühlung wird Salpetersäure zugetropft. Das ausgefallene Nitrat wird abgesaugt und getrocknet. Man erhält 15 g α-(1,2,4-Triazolyl)-acetophenonoxim-O-(4-chlorbenzyl)-ethernitrat vom Schmp. 130°C (Zersetzung).

In entsprechender Weise erhält man die in Tabelle 2 aufgeführten α-(1,2,4-Triazolyl)- bzw. α-Imidazolyl-acetophenonoximether der Formel IV. Kristallin anfallende Rohprodukte

können aus Essigester/Ether umkristallisiert werden. Aus Ether lassen sich nach Belieben auch Hydrochloride und andere Säureadditionssalze ausfällen.

Tabelle 2

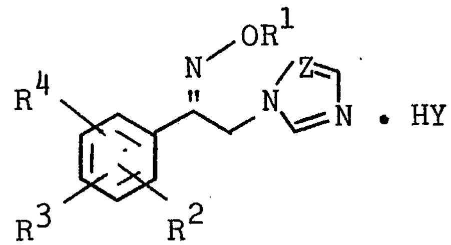

| $R^1$ | $R^2$ | $R^3$ | $R^4$ | $Y^{\ominus}$ | Z | Schmp. (°C) |
|---|---|---|---|---|---|---|
| $CH_3$ | 2-Cl | 4-Cl | H | Cl | N | 141 |
| $C_2H_5$ | 2-Cl | 4-Cl | H | Cl | N | 159 |
| Allyl | 2-Cl | 4-Cl | H | $NO_3$ | N | 115 |
| Propargyl | 2-Cl | 4-Cl | H | $NO_3$ | N | 108 |
| 2,4-Dichlorbenzyl | 2-Cl | 4-Cl | H | Cl | N | 173 |
| 4-Fluorbenzyl | 2-Cl | 4-Cl | H | | N | Öl |
| 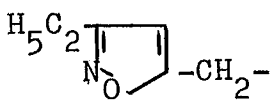 | 2-Cl | 4-Cl | H | | N | Öl |
| 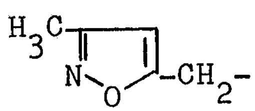 | 2-Cl | 4-Cl | H | | N | Öl |
| 2,4-Dichlorbenzyl | 4-Br | H | H | Cl | N | 168-170 |
| 2,4-Dichlorbenzyl | 4-Cl | H | H | Cl | N | 127 |
| $CH_3$ | 4-Cl | H | H | Cl | N | 156-158 |
| $CH_3$ | 4-Br | H | H | Cl | N | 157-169 |
| 4-Chlorbenzyl | 4-Br | H | H | Cl | N | 167-169 |

Tabelle 2 (Fortsetzung)

| $R^1$ | $R^2$ | $R^3$ | $R^4$ | $Y^{\ominus}$ | Z | Schmp. (°C) |
|---|---|---|---|---|---|---|
| Propargyl | 4-Br | H | H | | N | 83-84 |
| Allyl | 4-Br | H | H | | N | 70-71 |
| 2,4-Dichlorbenzyl | 4-tert.-$C_4H_9$ | H | H | $NO_3$ | N | 150-200 |
| $CH_3$ | 4-Cl | H | H | $NO_3$ | N | 169-172 |
| Allyl | 4-Cl | H | H | | N | 44-46 |
| Allyl | 4-Cl | H | H | Cl | N | 133-134 |
| $CH_2CN$ | 4-Cl | H | H | Cl | N | 167-170 |
| Propargyl | 4-Cl | H | H | Cl | N | 96-98 |
| $CH_2CO_2C_2H_5$ | 4-Cl | H | H | Cl | N | 133-135 |
| Propargyl | 2-Cl | 4-Cl | H | | N | öl |
| $CH_3$ | 2-Cl | 4-Cl | H | | N | öl |
| Benzyl | 2-Cl | 4-Cl | H | $NO_3$ | N | öl |
| Benzyl | 2-Cl | 4-Cl | H | Cl | N | öl |
| 2-Chlorbenzyl | 2-Cl | 4-Cl | H | Cl | N | öl |
| Allyl | 2-Cl | 4-Cl | H | | N | öl |
| 4-Chlorbenzyl | 2-Cl | 4-Cl | H | Cl | N | öl |
| 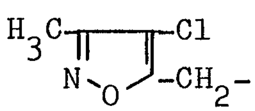 | 2-Cl | 4-Cl | H | | N | öl |
| n-Propyl | 2-Cl | 4-Cl | H | $NO_3$ | N | 100-102 |

Tabelle 2 (Fortsetzung)

| $R^1$ | $R^2$ | $R^3$ | $R^4$ | $Y^{\ominus}$ | Z | Schmp. (°C) |
|---|---|---|---|---|---|---|
| $C_2H_5$ | 2-Cl | 4-Cl | H | $NO_3$ | N | 103 |
| 3,4-Dichlorbenzyl | 2-Cl | 4-Cl | H | $NO_3$ | N | 119 |
| 3,4-Dichlorbenzyl | 2-Cl | 4-Cl | H | | N | Öl |
| $C_2H_5$ | 4-Cl | H | H | | N | 88-90 |
| n-Propyl | 4-Cl | H | H | $NO_3$ | N | 118-120 |
| $CH_3$ | $4-C_6H_5$ | H | H | $NO_3$ | N | 138-140 |
| $CH_3$ | 4-Cl | H | H | | N | 77-78 |
| $CH_3$ | H | H | H | $NO_3$ | N | 146 |
| $CH_3$ | 2-Cl | 4-Cl | H | $NO_3$ | CH | 150-152 |
| 2,4-Dichlorbenzyl | 4-Cl | H | H | Cl | CH | 127 |
| 2,4-Dichlorbenzyl | 4-Cl | H | H | | N | 91 |
| Methallyl | 2-Cl | 4-Cl | H | | N | |
| $CH_3$ | 2-Cl | 4-Cl | 6-Cl | | N | |

Bei der Synthese der Wirkstoffe der Formel IV kann man auch auf die Aufarbeitung der α-Chloracetophenonoximether I verzichten und die fungiziden α-Azolylacetophenonoximether in einer Eintopfreaktion aus den Phenacylchloriden der Formel II gewinnen, wie folgendes Beispiel zeigt:

Ein Gemisch aus 10 g O-Methylhydroxylammoniumhydrochlorid, 9 g Natriumacetat und 24,6 g 2,4,α-Trichloracetophenon und 200 ml Ethanol werden zwei Stunden unter Rückfluß erhitzt. Anschließend werden 22,8 g 1,2,4-Triazol, 35 g Natriumcarbonat, 200 ml Ethanol zugegeben. Man kocht drei Stunden unter Rückfluß, engt ein und nimmt mit Dichlormethan/Wasser auf. Die organische Phase wird mehrfach mit Wasser gewaschen, getrocknet und eingeengt.

Das Rohprodukt wird in Ether gelöst. Unter Eiskühlung wird mit Salpetersäure das Nitrat gefällt. Man erhält 18 g α-(1,2,4-Triazolyl)-2,4-dichlor-acetophenonoxim-O-methylethernitrat vom Schmp. 150-152°C.

## Patentansprüche

1. α-Chloracetophenonoximether der Formel I

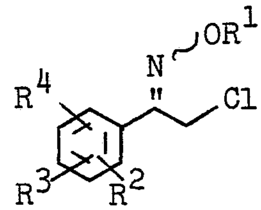

(I)

in der
$R^1$ Alkyl mit bis zu 4 Kohlenstoffatomen, Alkenyl mit bis zu 5 Kohlenstoffatomen, Alkinyl mit bis zu 4 Kohlenstoffatomen, Cycloalkyl mit bis zu 6 Kohlenstoffatomen, wobei der Alkyl-, Alkenyl-, Alkinyl- und der Cycloalkylrest jeweils durch 1 bis 3 Halogenatome substituiert sein können, gegebenenfalls durch Halogen, Alkyl oder Trifluormethyl einfach oder mehrfach im Arylrest substituiertes Aralkyl, gegebenenfalls durch Alkyl oder Halogen substituiertes Heteroarylalkyl sowie Cyanmethyl oder Alkoxycarbonylalkyl und $R^2$, $R^3$, $R^4$ unabhängig voneinander Wasserstoff, Halogen, Alkyl oder Alkoxy mit jeweils bis zu 4 Kohlenstoffatomen, Alkylthio mit bis zu 6 Kohlenstoffatomen, gegebenenfalls durch Halogen substituiertes Aryl oder Aralkyl, Aryloxy oder Arylthio bedeuten und zwei der Reste $R^2$, $R^3$, $R^4$ zusammen einen alicyclischen oder heterocyclischen Ring bilden können, an den ein weiterer aromatischer Ring ankondensiert sein kann.

2. Verfahren zur Herstellung von α-Chloracetophenonoximethern der Formel I, dadurch gekennzeichnet, daß man ein Phenacylchlorid der Formel II

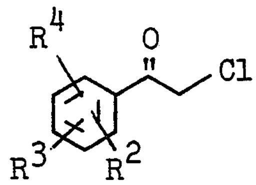

(II),

in der
$R^2$, $R^3$, $R^4$ die im Anspruch 1 genannten Bedeutungen haben, mit einem Hydroxylamin der Formel III

$H_2NOR^1$ (III),

in der
$R^1$ die im Anspruch 1 genannten Bedeutungen hat, oder einem Säureadditionssalz eines Hydroxylamins der Formel III in einem unter den Reaktionsbedingungen inerten organischen Lösungsmittel bei einer Temperatur im Bereich zwischen 20 und 120°C umsetzt.

0020970

Europäisches Patentamt

# EUROPÄISCHER RECHERCHENBERICHT

Nummer der Anmeldung
EP 80 10 2556

| EINSCHLÄGIGE DOKUMENTE | | | KLASSIFIKATION DER ANMELDUNG (Int.Cl. 3) |
|---|---|---|---|
| Kategorie | Kennzeichnung des Dokuments mit Angabe, soweit erforderlich, der maßgeblichen Teile | betrifft Anspruch | |
| D | THE JOURNAL OF ORGANIC CHEMISTRY, Band 36, Nr. 22, 5. November 1971, Seiten 3467-3469 Washington D.C., U.S.A. S.Y. CHU et al.: "Preparation of 2-Alkoxyiminoalkyl Bromides by the Bromination of O-Alkyl Oximes with N-Bromosuccinimide" * Tabelle, Seite 3468 * ---- | 1,2 | C 07 C 131/00// C 07 D 233/61 249/08 413/12 |

RECHERCHIERTE SACHGEBIETE (Int. Cl. 3)

C 07 C 131/00

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 11-09-1980 | GAUTIER |